# EUROPEAN PATENT SPECIFICATION

(11) **EP 4 031 024 B1**
(45) Date of publication and mention of the grant of the patent: **25.09.2024**
(21) Application number: 20864069.8
(22) Date of filing: 21.08.2020
(51) Int. Cl.: A61B 17/12, A61B 17/3205, A61B 17/3209

(54) **DEVICE FOR ATTACHING A THREAD LOOP AROUND A SKIN TAG**
VORRICHTUNG ZUM ANBRINGEN EINER FADENSCHLINGE UM EIN HAUTANHÄNGSEL
DISPOSITIF DE FIXATION D'UNE BOUCLE DE FIL AUTOUR D'UN FIBROME MOU

(30) Priority: 11.09.2019 SE 1930292
(43) Date of publication of application: 27.07.2022
(73) Proprietor: Scandinavian Health Trade AB, 17274 Sundbyberg (SE)
(72) Inventor: EKLUND, Gustaf, 22100 Mariehamn (FI); WIDLUND, David, 17274 Sundbyberg (SE)
(74) Representative: Bergenstråhle & Partners AB
(86) International application number: PCT/SE2020/050804
(87) International publication number: WO 2021/049989

(56) References cited:
- JP-A- 2016 007 246
- JP-A- H0 898 840
- US-A- 5 405 354
- US-A1- 2006 253 128
- US-A1- 2009 005 792
- US-A1- 2017 245 861
- US-A1- 2017 273 705
- US-A1- 2018 092 646
- US-A1- 2019 000 497
- No further relevant documents disclosed

## Description

### TECHNICAL FIELD

The present invention relates to a device being used to attach a contracted thread loop around an undesirable skin tag, whereby the skin tag is strangled, dies and falls off after a time.

### PRIOR ART

Skin tags are here used as the name of the skin growths which we can all get a bit here and there on the body, usually in the face, the neck, arm pits or groins. These skin tags are also called fibroma (acrochorda) and consist of connective tissue cells and collagen. They can be removed surgically by being cut off, burned away usually by means of alternating current, or by means of the application of active specific liquids. Skin tags are harmless but can be perceived as unpleasant. In this specification the term skin tag is also meant to include teardrop-shaped birthmarks as well as old age warts.

Prior art also includes mechanical devices where a user can choke the skin tag by means of a wire snare which is positioned around the base of the skin tag or by means of a loop being applied around the skin tag so that it, due to lack of nutrition, dies and falls off the skin. Such known technology is disclosed in, for example, the patent specification US 2017273705. In this text, a tool is reported in which a snare is tightened around a skin tag. A user pulls a ring connected to the snare. Due to this construction, the tool is not so convenient for being handled with only one hand, whereby it is not so well suited for a user who uses the tool on his/her own body. Furthermore, fitting the wire for use of the tool is not straightforward.

Another example of the prior art is disclosed in the publication US 2018092646. Here, an aid is described which comprises a thread snare which is wrapped around a skin tag. The thread has loose ends by means of which a user tightens the snare. This kind of aid is also not the easiest to handle for a user, especially when a user uses the aid for removing a skin tag on his own body.

As a further example of prior art, reference is made to the publication GB 2322802. In this is described a tool in which a loop of an elastic material is used being applied around the skin tag. In this case the loop is applied around the skin tag and passed over it by pushing the tool with its front part against and around the skin tag (i.e. against the skin on which the skin tag is located), after which the elastic loop is pressed over and around the skin tag.

### DESCRIPTION OF THE INVENTION

The device according to one aspect of the invention consists of a tool for applying a thread loop around a skin tag, the tool comprising a hollow cylindrical handle with a piston being arranged to run inside the handle, the piston having a first end which in a base position for the piston abuts against an opening in a front end of the handle. A thumb grip is attached to the piston and via a slot in the handle the piston is arranged accessible from the outside of the handle whereby the piston is displaceable from its base position along the interior of the handle. The piston has at a first end a carrier to which an inner end of a thread cartridge is arranged to be connected. A thread loop is arranged to be laid around the skin tag at an outer end of the thread cartridge. A displacement of the piston from the base position by means of the thumb grip concentrates the thread loop around the tag, when the piston with its carrier is displaced backwards from the base position of the piston and thereby stretches the thread when the thread of the thread cartridge is attached to the carrier. The inner end of the thread cartridge is arranged to be detached from the carrier when the handle is tilted in relation to the longitudinal direction of the thread cartridge, thereby slackening the thread, after which the handle is removed from the thread cartridge. The loop of the thread cartridge is then tightened around the skin tag and stops the supply of nutrients to it. The thread cartridge is left at the skin tag and falls off when the skin tag eventually loosens from the skin. It should be mentioned here that the thread cartridge is intended to form part of the device.

It should be mentioned here that the device is arranged to apply a tightened thread loop around a skin tag which has a narrow base so that the thread loop can be effectively applied around the skin tag without sliding away from it. For this reason, the device should be used only for the removal of skin tags (acrochorda) including also teardrop-shaped birthmarks and old age warts (seborrheic keratosis).

A spring inside the handle abuts a rear wall of the handle and presses against the other end of the piston so that the piston is pressed with a predetermined force against the opening in the front end of the handle. In this way a counterforce can be sensed by the user when the user withdraws the piston by moving the thumb grip backwards to tension the thread loop.

The thread cartridge is a pre-formed part which comprises a thread which at its front end has a tightenable loop when the rear part of the thread is pulled through a loop knot in front of which the loop is formed. At its rear end, the thread is provided with a knob being attached to the end of the thread. This knob is placed inside the wall of the carrier, whereby the thread is pulled into the handle when the piston is moved backwards from its base position. Around the thread, between the loop in its front part and the knob in its rear part, a hollow cylinder is arranged, where a through-hole is arranged along its axis. The thread of the thread cartridge can be displaced longitudinally along the through hole.

The carrier in the piston is arranged with a slot in its front wall. The purpose of this slot when loading the tool with the thread cartridge is to be able to insert the thread knob behind the wall of the carrier and to ensure that the thread can be advanced inside the slot and stretched mainly along the longitudinal axis of the handle in front of the handle.

The handle is at the far end of its front end equipped with a clamp with wings that has a longitudinal extension along the handle. When the thread cartridge is loaded to the handle and its piston, the cylinder is pressed down between said wings and becoming hold in position by the wings. Furthermore, a lug is formed next to the clamp behind it, whereby the cylinder cannot slide backwards when tensioning the thread in the thread cartridge when the piston is moved backwards, since the cylinder is stopped by the lug. As a result, each movement backwards of the thumb grip will bring the piston with its carrier backwards and then tighten the loop by pulling thread inwards into the handle.

The device has characteristics according to the features specified in claim 1. Further embodiments of the invention are presented in the dependent claims.

### BRIEF DESCRIPTION OF THE DRAWINGS

Figure 1 schematically shows a plan view of the device according to one aspect of the invention.
Figure 2 depicts in a perspective view a thread cartridge intended for connection to the tool when using it for its purpose.
Figure 3 shows a longitudinal section of the tool according to figure 1.
Figure 4 shows details of the device and their mutual positions in relation to each other.
Figure 5 illustrates the front part of the tool with its thread cartridge connected.
Figure 6 shows a plan view of a thread cartridge and a longitudinal section through the thread cartridge.
Figure 7 illustrates how the thread cartridge is released from the handle by moving the thumb grip forward and thereby slackening the thread so that it can slide out of the gap in the front of the carrier.

### DESCRIPTION OF EMBODIMENTS

In the following, a number of embodiments of the invention are described with reference to the accompanying drawings. The drawings show only schematically the principle of the device and do not claim to show to any scale any proportions between different elements thereof.

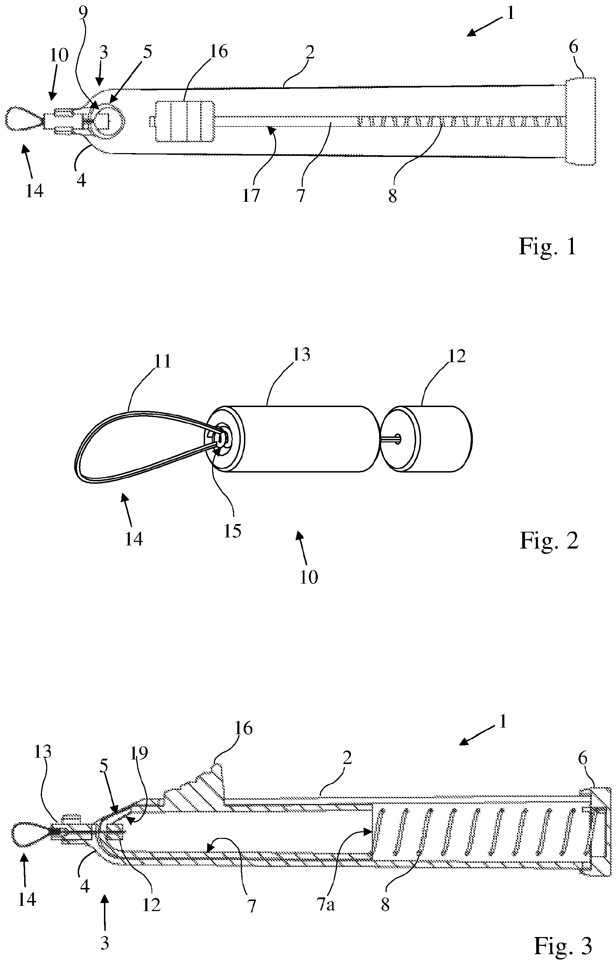

By reference to Figure 1, the tool 1 according to the aspect of the invention is shown. The tool has a handle 2 which consists of a hollow cylindrical body. Cylindrical means any type of cylinder that has a cross section with a closed curve. In the simplest embodiment, the handle 2 is circular-cylindrical. At its front end 3, the handle has a front wall 4 with an opening 5 which is an oblique cut-out across the handle housing next to the central longitudinal axis of the handle. At the rear end of the handle 2 this has a rear wall 6. The handle 2 has inside a cylindrical longitudinal cavity with preferably the same shape as the outside of the handle. A piston 7 is arranged in this cavity. This piston is arranged to have an external shape corresponding to the shape of the cavity, wherein the piston 7 can run longitudinally inside the handle 2. The piston 7 can be solid or designed hollow in a corresponding manner as the handle. The piston 7 is shorter than the cylindrical cavity of the handle. Between the piston 7 and the rear wall 6 of the handle, a spring 8, for example a helical spring, is arranged. The piston 7 is thus displaceable from a base position, where the first end 9 of the piston abuts against the front wall 4 of the handle 2, to a position rearwards where the spring 8 is completely compressed. The spring 8 abuts against a second end 7a, i.e. towards the rear end of the piston 7.

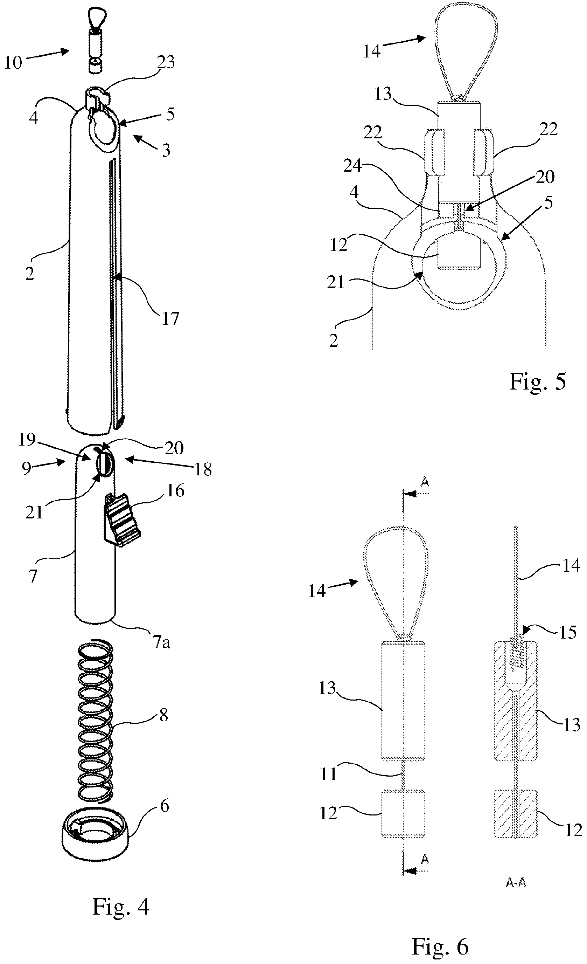

The thread cartridge 10 of the device is shown in detail in Figure 2. It appears that the thread cartridge has a thread 11 which is attached to a knob 12 at the rear. The thread 11 can run freely in a thread channel longitudinally through the hollow cylinder 13. At the front the thread 11 is formed into a tightenable loop 14. The loop knot 15 for the loop is glimpsed at the front of the cylinder 13. The loop knot 15 cannot be pulled into the cylinder 13, since a constriction (see figure 6) inside the thread channel in the cylinder prevents this. Thus, if the knob 12 is moved backwards while the cylinder 13 is held still, the loop 14 will be tightened against the loop knot 15.

The piston 7 is clearly shown in figure 4. Here it is shown with a thumb grip 16. This thumb grip has closest to the body of the piston 7 an elongate bracket which can run along an elongate gap 17 along the housing of the handle 2 in its longitudinal direction, the thumb grip thus being located on the outside of the handle 2 housing. Thus, when the piston 7 is located inside the cavity of the handle 2, the piston 7 can be moved by means of the thumb grip 16 along the gap 17 backwards against the spring 8 and compress this, whereby a counterforce against the movement of the thumb grip is achieved. At the front of the piston 7 at its front end, a carrier 18 is arranged. This carrier 18 consists of a wall 19 which comprises an elongate slot 20 which opens into a hole 21 in the wall 19 of the piston. When loading a thread cartridge 10 in the handle 2, the knob 12 is inserted through the hole 21 so that the knob 12 ends up behind the wall 19 while the thread is lowered into the gap 20, whereby the thread 11 can be pulled backwards and into the handle 2 when the piston 7 is moved backwards with the thumb grip 16.

The loading of a thread cartridge 10 to the handle 2 is most clearly shown in figure 5. The knob 12 of the thread cartridge is inserted through the hole 21 so that the knob 12 ends up behind the wall 19 in the piston 7 carrier 18. The thread in front of the knob 12 is lowered into the slot 20. The cylinder 13 is pressed down between the wings 22 at the foremost part of the handle 2. The wings 22 thereby form a clamp 23 which holds the cylinder 13 in position when a thread cartridge is loaded to the handle. Here it is also ensured that the rear wall of the cylinder 13 is arranged in front of an abutment in the form of a lug 24. As a result, the cylinder 13 surrounding the thread 11 cannot be moved backwards when the piston 7 carrier 18 stretches the thread while the piston 7 is displaced backwards by means of the thumb grip 16.

After charging a thread cartridge 10 to the handle 2, a pre-formed thread loop 14 is brought in over a skin tag to be removed from the skin of a client. This can of course also be done by the client himself. The thread loop 14 is laid around the skin tag at its base. After this the thumb grip 16 is displaced backwards along the handle. As a result, as shown above, the thread loop will be tightened around the skin tag. An advantage of this tool is that it can be handled with just one hand by one user.

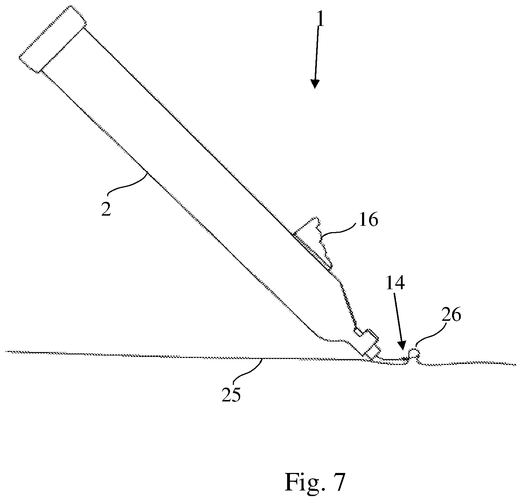

In Figure 7 it is shown how the thread cartridge 10 is detached from the handle 2. By the user releasing the grip on the handle 2, the thread 11 in the thread cartridge is slackened, since the spring 8 then pushes the piston 7 inside the handle 2 forwards. The user tilts at a large angle, as a suggestion 30 to 90 degrees, up the handle from the skin 25 and suitably presses the front part of the handle against the skin 25 on which the skin tag 26 is located and pulls the front part of the handle in the direction away from the skin tag. As a result, the cylinder 13 of the thread cartridge is detached from the clamp 23 and the thread 11 together with the knob 12 slides out of the carrier 18 due to the inclination of the handle in relation to the longitudinal direction of the thread 11 closest to the skin 25.

After detaching the handle from the thread cartridge, the thread cartridge 10 can remain at the skin tag 26 until it falls off. However, if so desired, the thread 11 can be cut off outside the loop knot 15 of the thread loop 14.

The thread cartridge can be marketed on the market in packages of a larger number with, for example, 5 or 10 thread cartridges per package.

## Claims

1. A device for applying a thread loop around a skin tag (26), the device comprising a thread cartridge (10) arranged to be connected to a tool (1) having a hollow cylindrical handle (2) with a piston (7) runningly arranged inside the handle, the piston (7) having a first end (9) which in a base position of the piston abuts to an opening (5) in a front end (3) of the handle, where the device comprises
- a thumb grip (16) is attached to the piston (7) and arranged accessible from the outside of the handle via a slot (17) in the handle (2) whereby the piston (7) is displaceable from its base position along the interior of the handle (2),
- the piston (7) has at its first end (9) a carrier (18) to which an inner end of said thread cartridge (10) is arranged to be connected,
- a thread loop (14) at an outer end of the thread cartridge (10) is arranged to be laid around the skin tag (26),
- wherein the piston (7) is configured to be displaced from the base position by means of the thumb grip (16) to pull together the thread loop (14) around the skin tag (26),
- the thread cartridge (10) is arranged to be detached from the carrier (18) by releasing the thumb grip (16) whereby the thread (11) of the thread cartridge can be detached from the carrier (18).

2. The device according to claim 1, wherein the thread cartridge (10) comprises a thread (11) having at its front end said tightenable thread loop (14) in front of a loop knot (15) and at its rear end a knob (12) intended to be fitted inside the wall (19) of the carrier (18), whereby the thread (11) is pulled into the handle (2) when the piston (7) is moved backwards from its base position, where further around the thread (11) between the thread loop (14) in its front part and the knob ( 12) in its rear part, a cylinder (13) with a through hole is arranged along its longitudinal axis, whereby the thread (11) in the thread cartridge (10) can be displaced longitudinally along said through hole.

3. The device according to claim 2, wherein the carrier (18) is arranged with a slot (20) in its front wall (19), wherein the thread (11) is lowered into the slot (20) and the knob (12) of the thread cartridge (10) arranged behind the carrier (18) wall when the thread cartridge is loaded to the tool (1).

4. The device according to claim 3, wherein the handle (2) outermost of its front end (3) is equipped with a clamp (23) with wings (22) and wherein the cylinder (13) of the thread cartridge (10) is pressed into the clamp (23) between said wings (22) when the thread cartridge (10) is loaded to the handle (2) and its piston (7) and where further a lug (24) is formed next to the clamp (23) behind it, whereby the cylinder (13) cannot slide backwards when tensioning the thread (11) in the thread cartridge (10) when the piston (7) is moved backwards because a rearward movement of the cylinder is then stopped by the lug (24).

5. The device according to claim 4, wherein a spring (8) is arranged inside the handle (2), wherein said spring abuts against a rear wall (6) of the handle and thereby presses against the second end (7a) of the piston so that the piston is pressed with a predetermined force against the opening (5) at the front end of the handle, whereby a counterforce can be sensed by a user who displaces the thumb grip backwards when moving the piston backwards for tensioning the thread loop (14).

6. The device according to claim 5, wherein the thumb grip (16) of the piston (7) has an elongate bracket which is displaceable in an elongate slot (17) along a casing of the handle (2) in its longitudinal direction, whereby the piston (7) is displaceable by means of the thumb grip (16) along the slot (17) backwards towards the spring (8), furthermore the piston (7) has at its front end said carrier (18) arranged where this carrier (18) consists of the wall (19) which comprises said slot (20) which opens into a hole (21) in the wall (19) of the piston.

## Patentansprüche

1. Vorrichtung zum Anbringen einer Fadenschlinge um ein Hautanhängsel (26), wobei die Vorrichtung eine Fadenpatrone (10) umfasst, die angeordnet ist, um mit einem Werkzeug (1) verbunden zu werden, das einen hohlen zylindrischen Griff (2) mit einem Kolben (7) aufweist, der innerhalb des Griffs laufend angeordnet ist, wobei der Kolben (7) ein erstes Ende (9) aufweist, welches in einer Grundposition des Kolbens an einer Öffnung (5) in einem vorderen Ende (3) des Griffs anliegt, wobei die Vorrichtung umfasst
- eine Daumenauflage (16) ist an dem Kolben (7) befestigt und von der Außenseite des Griffs über einen Schlitz (17) in dem Griff (2) zugänglich angeordnet, wodurch der Kolben (7) aus seiner Grundposition entlang der Innenseite des Griffs (2) verschiebbar ist,
- der Kolben (7) weist an seinem ersten Ende (9) einen Träger (18) auf, an welchem ein inneres Ende der Fadenpatrone (10) angeordnet ist, um verbunden zu werden,
- eine Fadenschlinge (14) an einem äußeren Ende der Fadenpatrone (10) ist angeordnet, um das Hautanhängsel (26) herum angeordnet zu werden,
- wobei der Kolben (7) konfiguriert ist, um anhand der Daumenauflage (16) aus der Grundposition verschoben zu werden, um die Fadenschlinge (14) um das Hautanhängsel (26) herum zusammenzuziehen,
- die Fadenpatrone (10) ist angeordnet, um durch Lösen der Daumenauflage (16) von dem Träger (18) abgelöst zu werden, wobei der Faden (11) der Fadenpatrone von dem Träger (18) abgelöst werden kann.

2. Vorrichtung nach Anspruch 1, wobei die Fadenpatrone (10) einen Faden (11) umfasst, welcher an seinem vorderen Ende die festziehbare Fadenschlinge (14) vor einem Schlingenknoten (15), und an seinem hinteren Ende einen Knopf (12) aufweist, welcher dazu bestimmt ist, in die Wand (19) des Trägers (18) eingesetzt zu werden, wodurch der Faden (11) in den Griff (2) gezogen wird, wenn der Kolben (7) aus seiner Grundposition nach hinten bewegt wird, wobei weiter um den Faden (11) herum, zwischen der Fadenschlinge (14) in seinem vorderen Teil und dem Knopf (12) in seinem hinteren Teil ein Zylinder (13) mit einem Durchgangsloch entlang seiner Längsachse angeordnet ist, wodurch der Faden (11) in der Fadenpatrone (10) entlang des Durchgangslochs in Längsrichtung verschoben werden kann.

3. Vorrichtung nach Anspruch 2, wobei der Träger (18) mit einem Schlitz (20) in seiner Vorderwand (19) angeordnet ist, wobei der Faden (11) in den Schlitz (20) abgesenkt ist und der Knopf (12) der Fadenpatrone (10) hinter der Wand des Trägers (18) angeordnet ist, wenn die Fadenpatrone in das Werkzeug (1) geladen ist.

4. Vorrichtung nach Anspruch 3, wobei der Griff (2) ganz außen an seinem vorderen Ende (3) mit einer Klemme (23) mit Flügeln (22) ausgestattet ist, und wobei der Zylinder (13) der Fadenpatrone (10) in die Klemme (23) zwischen den Flügeln (22) gedrückt wird, wenn die Fadenpatrone (10) in den Griff (2) und seinen Kolben (7) geladen wird, und wobei weiter neben der Klemme (23) dahinter eine Nase (24) gebildet ist, wodurch der Zylinder (13) beim Spannen des Fadens (11) in der Fadenpatrone (10) nicht nach hinten rutschen kann, wenn der Kolben (7) nach hinten bewegt wird, da eine Rückwärtsbewegung des Zylinders dann durch die Nase (24) gestoppt wird.

5. Vorrichtung nach Anspruch 4, wobei im Inneren des Griffs (2) eine Feder (8) angeordnet ist, wobei die Feder an einer Rückwand (6) des Griffs anliegt und dadurch gegen das zweite Ende (7a) des Kolbens drückt, sodass der Kolben mit einer vorbestimmten Kraft gegen die Öffnung (5) am vorderen Ende des Griffs gedrückt wird, wodurch beim Zurückbewegen des Kolbens zum Spannen der Fadenschlinge (14) durch einen Benutzer, der die Daumenauflage nach hinten verschiebt, eine Gegenkraft verspürt werden kann.

6. Vorrichtung nach Anspruch 5, wobei die Daumenauflage (16) des Kolbens (7) einen länglichen Bügel aufweist, der in einem länglichen Schlitz (17) entlang eines Gehäuses des Griffs (2) in ihrer Längsrichtung verschiebbar ist, wodurch der Kolben (7) anhand der Daumenauflage (16) entlang des Schlitzes (17) nach hinten in Richtung der Feder (8) verschiebbar ist, der Kolben (7) weiter an seinem vorderen Ende den Träger (18) angeordnet aufweist, wobei dieser Träger (18) aus der Wand (19) besteht, welche den Schlitz (20) umfasst, welcher sich in ein Loch (21) in der Wand (19) des Kolbens öffnet.

## Revendications

1. Dispositif pour appliquer une boucle de fil autour d'une fibrome mou (26), le dispositif comprenant une cartouche de fil (10) agencée pour être reliée à un outil (1) présentant une poignée cylindrique creuse (2) avec un piston (7) agencé de manière mobile à l'intérieur de la poignée, le piston (7) présentant une première extrémité (9) qui, dans une position de base du piston, vient en butée contre une ouverture (5) dans une extrémité avant (3) de la poignée, dans lequel le dispositif comprend
- une prise de pouce (16) qui est fixée au piston (7) et agencée de manière accessible depuis l'extérieur de la poignée par le biais d'une fente (17) dans la poignée (2), selon lequel le piston (7) peut être déplacé depuis sa position de base le long de l'intérieur de la poignée (2),
- le piston (7) présente, au niveau de sa première extrémité (9), un support (18) auquel une extrémité interne de ladite cartouche de fil (10) est agencée pour être reliée,
- une boucle de fil (14) au niveau d'une extrémité externe de la cartouche de fil (10) qui est agencée pour être disposée autour du fibrome mou (26),
- dans lequel le piston (7) est configuré pour être déplacé depuis la position de base au moyen de la prise de pouce (16) pour rassembler la boucle de fil (14) autour du fibrome mou (26),
- la cartouche de fil (10) est agencée pour être détachée du support (18) en relâchant la prise de pouce (16), selon lequel le fil (11) de la cartouche de fil peut être détaché du support (18).

2. Dispositif selon la revendication 1, dans lequel la cartouche de fil (10) comprend un fil (11) présentant, au niveau de son extrémité avant, ladite boucle de fil pouvant être serrée (14) devant un noeud de boucle (15) et, au niveau de son extrémité arrière, un bouton (12) destiné à être monté à l'intérieur de la paroi (19) du support (18), selon lequel le fil (11) est tiré dans la poignée (2) lorsque le piston (7) est déplacé vers l'arrière depuis sa position de base, dans lequel, plus loin autour du fil (11) entre la boucle de fil (14) dans sa partie avant et le bouton (12) dans sa partie arrière, un cylindre (13) avec un trou traversant est agencé le long de son axe longitudinal, selon lequel le fil (11) dans la cartouche de fil (10) peut être déplacé longitudinalement le long dudit trou traversant.

3. Dispositif selon la revendication 2, dans lequel le support (18) est agencé avec une fente (20) dans sa paroi avant (19), dans lequel le fil (11) est abaissé dans la fente (20) et le bouton (12) de la cartouche de fil (10) est agencé derrière la paroi de support (18) lorsque la cartouche de fil est chargée sur l'outil (1).

4. Dispositif selon la revendication 3, dans lequel la poignée (2) la plus à l'extérieur de son extrémité avant (3) est équipée d'une pince (23) avec des ailes (22) et dans lequel le cylindre (13) de la cartouche de fil (10) est pressé dans la pince (23) entre lesdites ailes (22) lorsque la cartouche de fil (10) est chargée sur la poignée (2) et son piston (7) et dans lequel, en outre, un ergot (24) est formé à côté de la pince (23) derrière celle-ci, selon lequel le cylindre (13) ne peut pas glisser vers l'arrière lors d'une mise en tension du fil (11) dans la cartouche de fil (10) lorsque le piston (7) est déplacé vers l'arrière parce qu'un déplacement vers l'arrière du cylindre est alors arrêté par l'ergot (24 ).

5. Dispositif selon la revendication 4, dans lequel un ressort (8) est agencé à l'intérieur de la poignée (2), dans lequel ledit ressort vient en butée contre une paroi arrière (6) de la poignée et, de ce fait, appuie contre la seconde extrémité (7a) du piston de telle sorte que le piston soit pressé avec une force prédéterminée contre l'ouverture (5) au niveau de l'extrémité avant de la poignée, selon lequel une force contraire peut être détectée par un utilisateur qui déplace la prise de pouce vers l'arrière lors du déplacement du piston vers l'arrière pour mettre en tension la boucle de fil (14).

6. Dispositif selon la revendication 5, dans lequel la prise de pouce (16) du piston (7) présente un support allongé qui peut être déplacé dans une fente allongée (17) le long d'un boîtier de la poignée (2) dans sa direction longitudinale, selon lequel le piston (7) peut être déplacé au moyen de la prise de pouce (16) le long de la fente (17) vers l'arrière vers le ressort (8), en outre le piston (7) présente, au niveau de son extrémité avant, ledit support (18) agencé là où ce support (18) est composé de la paroi (19) qui comprend ladite fente (20) qui débouche dans un trou (21) dans la paroi (19) du piston.
